# EUROPÄISCHE PATENTSCHRIFT

(11) **EP 0 583 561 B1**
(45) Veröffentlichungstag und Bekanntmachung des Hinweises auf die Patenterteilung: **01.04.1998**
(21) Anmeldenummer: 93108826.4
(22) Anmeldetag: 02.06.1993
(51) Int. Cl.: C07D 207/34, C07D 275/02, C07D 401/04

(54) **Verfahren zur Herstellung von 1-unsubstituierten 3-Aminopyrrolen**
Process for the preparation of 1-unsubstituted 3-aminopyrroles
Procédé pour la préparation de 3-aminopyrroles 1-nonsubstitués

(30) Priorität: 20.08.1992 DE 4227479
(43) Veröffentlichungstag der Anmeldung: 23.02.1994
(73) Patentinhaber: ARZNEIMITTELWERK DRESDEN GmbH, D-01435 Radebeul (DE)
(72) Erfinder: Rolfs, Andreas, Dipl.-Chem., O-1090 Berlin (DE); Liebscher, Jürgen, Prof. Dr., O-Neuenhagen (DE); Unverferth, Klaus, Dr., O-8019 Dresden (DE); Faust, Gottfried, Dr.rer.nat., O-8122 Radebeul (DE)

(56) Entgegenhaltungen:
- EP-A- 0 431 371
- DD-A- 298 913
- DD-A- 298 914
- DD-A- 298 915
- DD-A- 298 916
- DD-A- 298 917
- DD-A- 298 918
- CHEMICAL ABSTRACTS, vol. 103, no. 15, 14. Oktober 1985, Columbus, Ohio, US; abstract no. 123423t, Seite 723 ;Spalte L ; & KHIM. GETEROTSIKL. SOEDIN. Bd. 5 , 1985 , BERLIN Seiten 628 - 630
- COMPREHENSIVE HETEROCYCLIC CHEMISTRY Bd. 4, Nr. 3 , 1984 , OXFORD Seite 144
- ANGEWANDTE CHEMIE Bd. 105, Nr. 5 , Mai 1993 , WEINHEIM Seiten 797 - 799 ANDREAS ROLFS ET.AL.

## Beschreibung

Die Erfindung betrifft ein Verfahren zur Herstellung von 1-unsubstiuierten 3-Aminopyrrolen.
Derartige Verbindungen besitzen Bedeutung für die Verwendung als Arzneistoffe oder Arzneimittel mit ZNS-Wirkung, vorzugsweise mit antikonvulsiven Eigenschaften. Die Erfindung ist in der chemischen und pharmazeutischen Industrie einsetzbar.

Es ist bekannt, daß sich 3-Morpholino-pyrrol-2-carbonsäureester aus 3-Aminothioacrylsäureamiden und Glycinestern herstellen lassen (A. Knoll, J. Liebscher Khim. Geterotsikl. Soedin. 1985, 628). Es wurde beansprucht, daß 3-Aminopyrrole mit pharmakologischer Wirkung, insbesondere mit ZNS-aktiven Eigenschaften nach verschiedenen Verfahren aus offenkettigen Vorläufern hergestellt werden können (EP-OS-0431371). Weiterhin sind 3-Aminopyrrole, die in 4-Position Aminocarbonyl- (DE 2605419) oder Carbonylgruppen (US 4198502) tragen, als ZNS-wirksame Substanzen eingestuft worden. Die bekannten Verfahren besitzen den Nachteil, daß entweder Mehrstufenprozesse notwendig sind oder gesundheitsschädliche Nebenprodukte, wie beispielsweise Alkylmercaptane gebildet werden. Es ist bekannt, daß N-substituierte 1,2-Thiazoliumsalze Ringtransformationsreaktionen zu Thiophenen eingehen können (C. W. Bird, G. W. H. Cheeseman in Comprehensive Heterocyclic Chemistry, Pergamon Press, Oxford, New York,Toronto, Sydney, Paris, Frankfurt, 1984, Vol. 4, S. 144). Schließlich ist bekannt, daß Isothiazoliumsalze in Gegenwart von Basen leicht den 2-ständigen Substituenten abspalten (S.Rajappa, G B. Advani, R. Sreenivasan Indian J. Chem. B,15 (1977) 848).

Aufgabe der Erfindung ist es, ein neues Verfahren zur Herstellung von pharmakologisch wirksamen 1-unsubstituierten 3-Aminopyrrolen zu entwickeln, das es gestattet, derartige Verbindungen in einem Einstufenprozeß herzustellen, ohne daß gesundheitsschädliche Nebenprodukte entstehen.

Erfindungsgemäß wird diese Aufgabe dadurch gelöst, daß 1-unsubstituierte 3-Aminopyrrole der
allgemeinen Formel I, 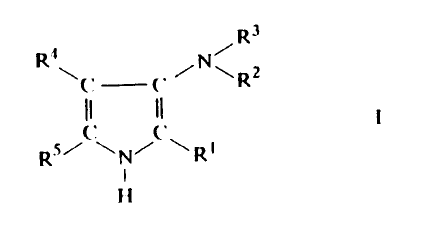 in der R1 Wasserstoff, Alkyl, Alkenyl, Aryl, Hetaryl, Nitro, Cyano, Acyl, Alkoxycarbonyl, Aminocarbonyl, Alkoxycarbonyl, Aryloxycarbonyl oder eine Sulfonylgruppe, R2 und R3 gleich oder verschieden Wasserstoff, Aryl- oder Alkylreste, die auch durch Heteroatome substituiert sein können, R2 und R3 zusammmen eine Alkylbrücke, die auch Heteroatome wie Stickstoff, Sauerstoff oder Schwefel enthalten beziehungsweise substituiert sein kann, R4 und R5 gleich oder verschieden Wasserstoff, eine Alkoxycarbonyl-, eine Aminocarbonyl- oder eine Aminothiocarbonylgruppe, einen unsubstituierten oder substituierten Alkyl- oder Aryl- beziehungsweise Hetarylrest, darstellen, dadurch gekennzeichnet, daß ein 1,2-Thiazoliumsalz der allgemeinen Formel II 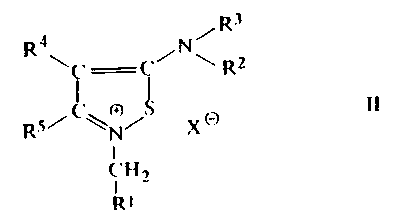 mit der für R1, R2, R3 beziehungsweise R2/R3, R4 und R5 erklärten Bedeutung und in der X^{⊖} für ein Säurerestanion, wie beispielsweise Halogenid, Perchlorat, Tetrafluoroborat, Hydrogensulfat, Sulfat, Hydroxid oder Triflat steht, vorteilhafterweise in Gegenwart einer Base ringtransformiert wird.
Als Base läßt sich beispielsweise ein tertiäres Amin, ein basischer N-Heteroaromat, ein Alkalihydrid, ein Alkaliamid, ein Alkalicarbonat, ein Alkalihydroxid oder ein Ionenaustauscher verwenden. Das erfindungsgemäße Verfahren gestattet es, 1-unsubstituierte 3-Aminopyrrole der allgemeinen Formel I in nur einem Reaktionsschritt zu synthetisieren, ohne daß gesundheitsschädliche Nebenprodukte, zum Beispiel Schwefelwasserstoff oder Mercaptane, gebildet werden. Die hergestellten Verbindungen besitzen eine hohe antikonvulsive Aktivität.

Überraschenderweise wird bei den Umsetzungen keine Abspaltung des 2-ständigen Substituenten der 1,2-Thiazoliumsalze der allgemeinen Formel II beobachtet. Das erfindungsgemäße Verfahren beschreibt eine prinzipiell neuartige Synthese für das Pyrrolgerüst, bei der dieser Heterocyclus durch Ringtransformation eines 1,2-Thiazols aufgebaut wird. Mit der Ringtransformation lassen sich auch solche Pyrrole darstellen, die mit den bisher bekannten Verfahren nicht zugänglich waren. Die Ausbeuten sind hoch und die Produkte können unkompliziert, meist durch einfaches Umkristallisieren gereinigt werden.
Die Erfindung soll nachstehend an einigen Ausführungsbeispielen näher erläutert werden.

Tabelle 1: 1-unsubstituierte 3-Aminopyrrole der allgemeinen Formel I

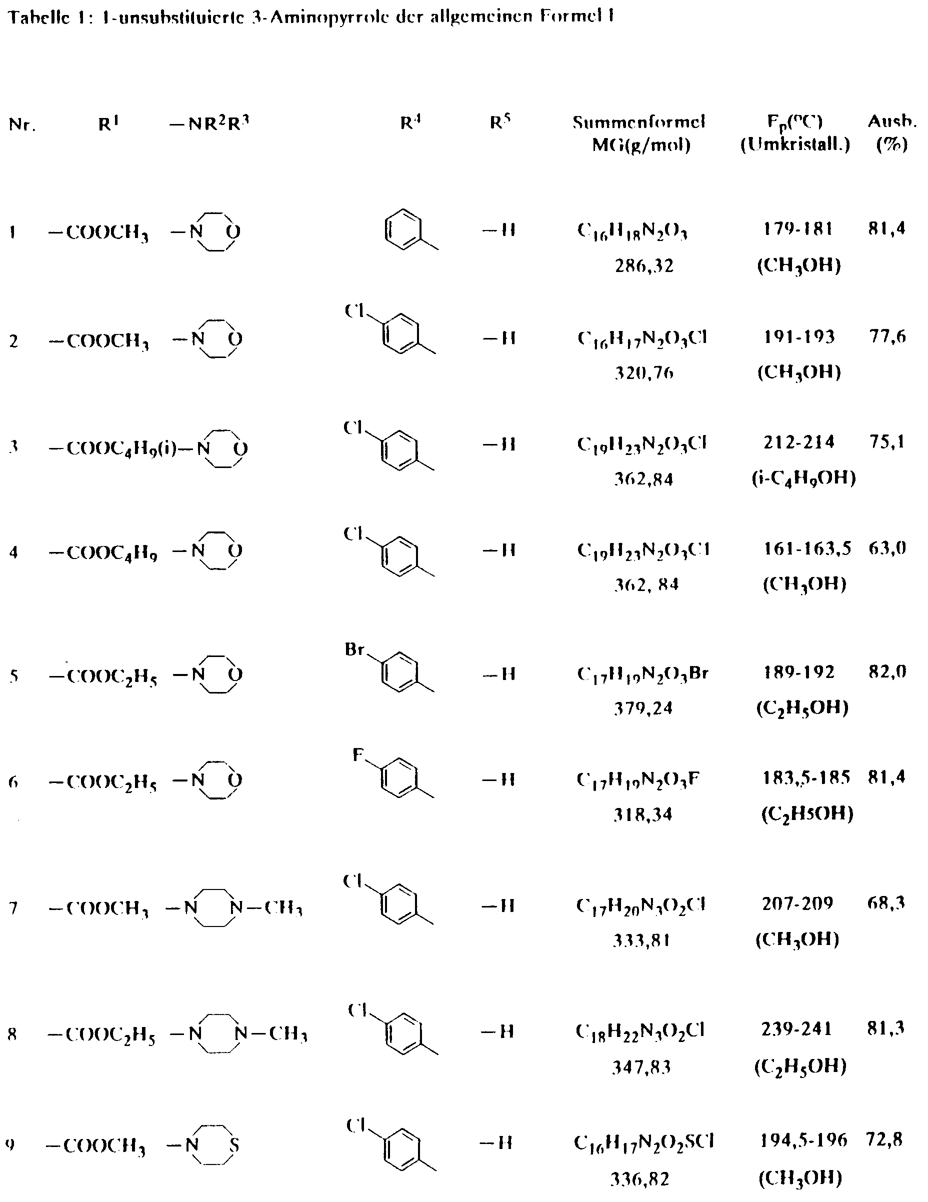

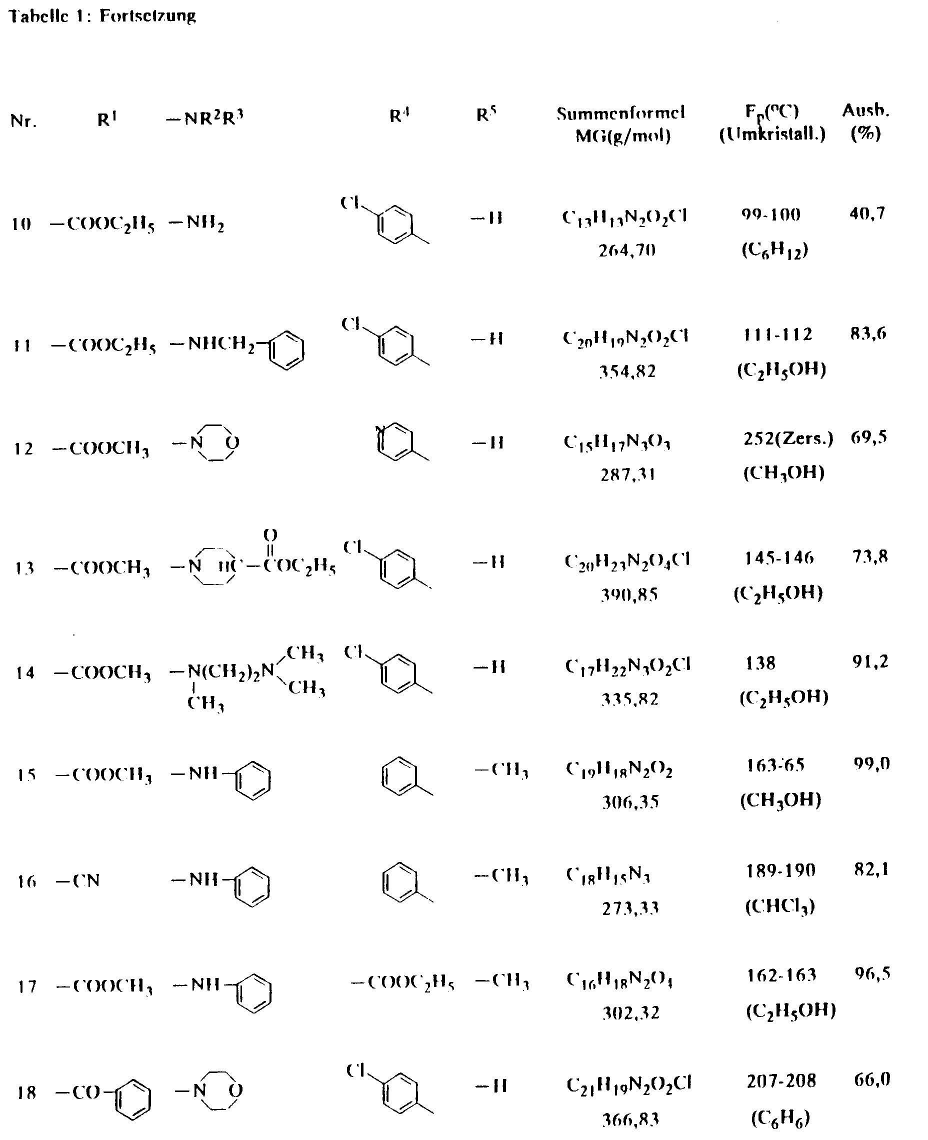

### Allgemeine Arbeisvorschrift zur Darstellung 1-unsubstituierter 3-Aminopyrrole der allgemeinen

### Formel I:

0,01 mol des 1,2-Thiazoliumsalzes der allgemeinen Formel II werden in 40-50 ml Ethanol, Methanol, Acetonitril oder Chloroform gelöst und mit einem leichten Überschuß an tertiärem Amin, N-Heteroaromat, Alkalihydroxid oder Alkalicarbonat versetzt und bis zu 5 min unter Rückfluß erhitzt. Der meist schon in der Hitze ausfallende größte Teil des freiwerdenden Schwefels wird abfiltriert. Die Mutterlauge wird auf 0 °C abgekühlt. Nach ca. 2-3 Stunden werden die ausgefallenen Kristalle der 3-Aminopyrrole der allgemeinen Formel I abgesaugt und aus dem entsprechenden Lösungsmittel umkristallisiert. Eine Ausbeuteerhöhung über die in Tabelle 1 angegebenen Werte kann durch Einengung der Mutterlaugen, Nachfällung mit Wasser oder eine säulenchromatographische Reinigung der Mutterlauge erfolgen.
Im Falle der Umsetzung der 1,2-Thiazoliumsalze mit Alkalihydrid fanden DMF, Dioxan oder Tetrahydrofuran als Lösungsmittel Verwendung. Die Aufarbeitung erfolgte hier durch Schütten der Reaktionsmischung auf Eiswasser, Filtration und anschließender Umkristallisation.

### Ausgewählte Arbeitsvorschriften zur Darstellung 1-unsubstituierter 3-Aminopyrrole:

### Beispiel 1

### 4-(4-Chlorphenyl)-3-(morpholin-1-yl)pyrrol-2-carbonsäure-n-butylester:

0,01 mol 4-(4-Chlorphenyl)-5-(morpholin-4-yl)-N-butyloxycarbonyl-methyl-1,2-thiazoliumbromid werden in 50 ml Ethanol gelöst und 0,012 mol Triethylamin in 5ml Ethanol zugetropft. Die Lösung wird unter Rühren zum Sieden erhitzt. Der ausfallende Schwefel wird heiß abfiltriert und die Mutterlauge mit 10 ml heißem Wasser versetzt. Die Lösung wird auf 0°C abgekühlt und die ausgefallenen Kristalle werden abgesaugt. Durch Lösen in Methylenchlorid wird noch anhaftender Schwefel entfernt. Die zur Trockne eingeengte Methylenchloridlosung wird mit Methanol aufgenommen und die ausfallenden Kristalle werden abgesaugt und getrocknet.
Ausbeute: 2,28g (63 % d. Theorie)
Elementaranalyse (ber./gef.): C: 62,89/62,62 H: 6,39/6,44 N: 7,72/7,80 Cl: 9,77/9,78

### Beispiel 2

### 4-(4-Chlorphenyl)-3-(4-methylpiperazin-1-yl)pyrrol-2-carbonsäu remethylester:

0,01 mol 4-(4-Chlorphenyl)-5-(4-methylpiperazin-1-yl)-N-methyloxycarbonylmethyl-1,2-thiazoliumhydroxid werden in 50 ml Methanol 5 min. unter Rückfluß erhitzt. Die Lösung wird auf 0°C abgekühlt, nach 2 Stunden werden die ausgefallenen Kristallen abgesaugt und aus Methanol umkristallisiert.
Ausbeute: 2,27g (68% d. Theorie)
Elementaranalyse (ber./gef.): C: 61,16/61,39 H: 6,04/5,90 N: 12,59/12,49 Cl: 10,62/10,61

### Beispiel 3

### 3-(Morpholin-4-yl)-4-(pyridin-4-yl)pyrrol-2-carbonsäuremethylester:

0,01 mol 5-(Morpholin-4-yl)-4-(pyridin-4-yl)-N-methyloxycarbonylmethyl-1,2-thiazoliumiodid werden in 40 ml einer 1n-ethanolischen Natronlauge 2 min. zum Sieden erhitzt. Die Lösung wird abgekühlt, die ausgefallenen Kristalle werden abgesaugt und anschließend durch Säulenchromatographie von anhaftendem Schwefel gereinigt.
Ausbeute: 2g (69,5% d. Theorie)
Elementaranalyse (ber./gef.): C: 62,70/62,89 H: 5,96/6,00 N: 14,63/14,67

### Beispiel 4

### 3-Benzylamino-4-(4-chlorphenyl)pyrrol-2-carbonsäureethylester:

0,01 mol 5-Benzylamino-4-(4-chlorphenyl)-N-ethyloxycarbonylmethyl-1,2-thiazoliumbromid werden in 40 ml einer wässrig-ethanolischen 0,01n-Kaliumcarbonatlösung 5 min. am Rückfluß gekocht.
Nach Abkühlung auf 0°C werden die Kristalle abgesaugt und aus Ethanol umkristallisiert.
Ausbeute: 2,97g (83,6% d.Theorie)
Elementaranalyse (ber./gef.): C: 67,69/67,54 H: 5,40/5,38 N:7,90/8,00 Cl: 9,99/10,13

### Antikonvulsive Wirkung 1-unsubstituierter 3-Aminopyrrole der allgemeinen Formel I:

### Maximaler Elektrokrampf (Maus)

### 3-(Morpholin-4-yl)-4-phenyl-pyrrol-2-carbonsäuremethylester:

Dosis: 1.10-3mol/kg i.p. = 100% Krampfhemmung

### 4-(4-Chlorphenyl)-3-(thiomorpholin-4-yl)pyrrol-2-carbonsäuremethylester:

Dosis: 5.10-4mol/kg i.p. = 80% Krampfhemmung

## Patentansprüche

1. Verfahren zur Herstellung von 1-unsubstituierten 3-Aminopyrrolen der allgemeinen Formel I, 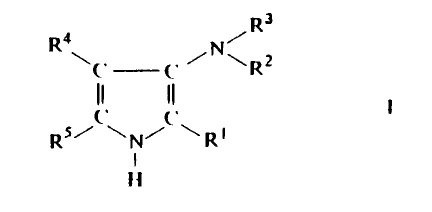 in der R1 Wasserstoff, Alkyl, Alkenyl, Aryl, Hetaryl, Nitro, Cyano, Acyl, Alkoxycarbonyl, Aminocarbonyl, Alkoxycarbonyl, Aryloxycarbonyl oder eine Sulfonylgruppe, R2 und R3 gleich oder verschieden Wasserstoff, Aryl- oder Alkylreste, die auch durch Heteroatome substituiert sein können, R2 und R3 zusammmen eine Alkylbrücke, die auch Heteroatome wie Stickstoff, Sauerstoff oder Schwefel enthalten beziehungsweise substituiert sein kann, R4 und R5 gleich oder verschieden Wasserstoff, eine Alkoxycarbonyl-, eine Aminocarbonyl- oder eine Aminothiocarbonylgruppe, einen unsubstituierten oder substituierten Alkyl- oder Aryl- beziehungsweise Hetarylrest, darstellen, dadurch gekennzeichnet, daß ein 1,2-Thiazoliumsalz der allgemeinen Formel II 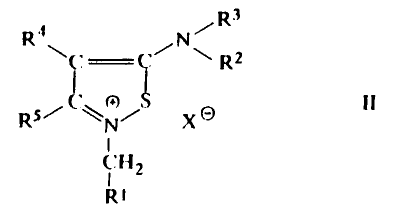 mit der für R1, R2, R3 beziehungsweise R2/R3, R4 und R5 erklärten Bedeutung und in der X^{⊖} für ein Säurerestanion, wie beispielsweise Halogenid, Perchlorat, Tetrafluoroborat, Hydrogensulfat, Sulfat, Hydroxid oder Triflat steht, vorteilhafterweise in Gegenwart einer Base ringtransformiert wird.

2. Verfahren nach Anspruch 1, dadurch gekennzeichnet, daß als Base beispielsweise ein tertiäres Amin, ein basischer N-Heteroaromat, ein Alkalihydrid, ein Alkaliamid, ein Alkalicarbonat, ein Alkalihydroxid oder ein Ionenaustauscher verwendet wird.

## Claims

1. Process for the preparation of 1-unsubstituted 3-aminopyrroles of the general formula I 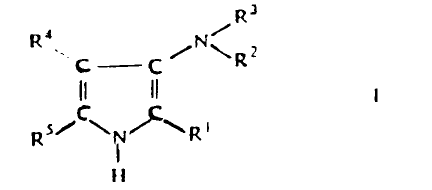 in which R¹ is hydrogen, alkyl, alkenyl, aryl, hetaryl, nitro, cyano, acyl, alkoxycarbonyl, aminocarbonyl, alkoxycarbonyl aryloxycarbonyl or a sulphonyl group, R² and R³, identically or differently, are hydrogen, aryl or alkyl radicals, which can also be substituted by heteroatoms, R² and R³ together are an alkyl bridge which can also contain heteroatoms such as nitrogen, oxygen or sulphur or can be substituted, R⁴ and R⁵, identically or differently, are hydrogen, an alkoxycarbonyl, an aminocarbonyl or an aminothiocarbonyl group, an unsubstituted or substituted alkyl or aryl radical or hetaryl radical, characterized in that a 1,2-thioazolium salt of the general formula II 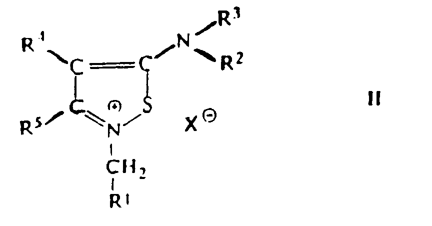 having the meaning explained for R¹, R², R³ or R²/R^{3,} R⁴ and R⁵ and in which X^{⊖} is an acid radical anion, such as, for example, halide, perchlorate, tetrafluoroborate, hydrogen sulphate, sulphate, hydroxide or triflate, is advantageously ring-transformed in the presence of a base.

2. Process according to Claim 1, characterized in that the base used is, for example, a tertiary amine, a basic N-heteroaromatic, an alkali metal hydride, an alkali metal amide, an alkali metal carbonate, an alkali metal hydroxide or an ion exchanger.

## Revendications

1. Procédé de préparation de 3-aminopyrroles non substitués en 1 de formule générale I 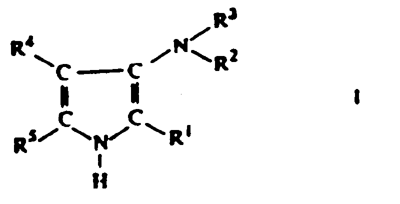 dans laquelle R1 représente l'hydrogène, un alkyle, un alcényle, un aryle, un hétéroaryle, un nitro, un cyano, un acyle, un alcoxycarbonyle, un aminocarbonyle, un aryloxycarbonyle ou un groupe sulfonyle, R2 et R3, identiques ou différents représentent l'hydrogène, des radicaux aryle ou alkyle qui peuvent être également substitués par des hétéroatomes, R2 et R3 représentent conjointement un pont alkyle, qui peut également contenir des hétéroatomes comme l'azote, l'oxygène, ou le soufre ou être substitué, R4 et R5, identiques ou différents, représentent l'hydrogène, un groupe alcoxycarbonyle, aminocarbonyle ou aminothiocarbonyle, un radical alkyle, aryle ou hétéroaryle non substitué ou substitué, caractérisé en ce qu'un sel de 1,2-thiazolium de formule générale II 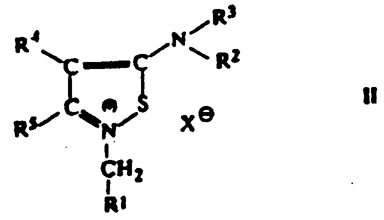 avec la signification donnée pour R1, R2, R3 respectivement R2/R3, R4 et R5, et dans laquelle X⁻représente un anion de radical acide, comme par exemple un halogénure, un perchlorate, un tétrafluoroborate, un hydrogénosulfate, un sulfate, un hydroxyde ou un triflate, est transformé au niveau du cycle avantageusement en présence d'une base.

2. Procédé selon la revendication 1, caractérisé en ce que l'on utilise, en tant que base, par exemple une amine tertiaire, un N-hétéroaromatique basique, un hydrure alcalin, un amidure alcalin, un carbonate alcalin, un hydroxyde alcalin ou un échangeur d'ions.
